Europäisches Patentamt

**European Patent Office**

**Office européen des brevets**

(11) Numéro de publication: **0 177 004 B1**

(12) **FASCICULE DE BREVET EUROPEEN**

(45) Date de publication de fascicule du brevet: **03.06.92**  (51) Int. Cl.5: **C03B 9/40**, G01N 21/90, G07C 3/14

(21) Numéro de dépôt: **85112366.1**

(22) Date de dépôt: **30.09.85**

(54) **Procédé et dispositif de contrôle sans contact d'objets fabriqués automatiquement à haute cadence.**

(30) Priorité: **02.10.84 FR 8415117**

(43) Date de publication de la demande:
**09.04.86 Bulletin 86/15**

(45) Mention de la délivrance du brevet:
**03.06.92 Bulletin 92/23**

(84) Etats contractants désignés:
**AT BE CH DE FR GB IT LI LU NL SE**

(56) Documents cités:
**EP-A- 0 059 572**
**EP-A- 0 059 575**
**GB-A- 2 087 549**
**GB-A- 2 135 452**

**GLASS TECHNOLOGY, vol. 22, no. 3, juin 1981, pages 139-140, Sheffield, GB; W. SANDERS: "Solid state optics for sidewall and dimensional inspection of glassware"**

(73) Titulaire: **VERRERIE DU LANGUEDOC ET CIE.**

**F-30310 Vergeze(FR)**

(72) Inventeur: **Leser, Jaques**
**Rue des Pins**
**F-34400 Lunel, Paris(FR)**

(74) Mandataire: **Casalonga, Axel et al**
**BUREAU D.A. CASALONGA - JOSSE Morassistrasse 8**
**W-8000 München 5(DE)**

Rank Xerox (UK) Business Services

## Description

La présente invention a pour objet un procédé et un dispositif permettant le contrôle sans contact d'objets fabriqués à haute cadence de manière automatique. L'invention s'applique en particulier à la fabrication d'objets en verre à haute température. Le procédé de l'invention peut permettre une intervention sur la commande automatique de la machine de fabrication en tenant compte de la mesure effectuée, pour améliorer la fabrication.

L'invention peut être utilisée par exemple pour améliorer la qualité de bouteilles en verre moulé fabriquées notamment par injection-soufflage.

La production de masse d'objets à haute cadence nécessite l'organisation de procédures de contrôle de qualité, efficaces et n'entraînant pas des frais excessifs. Ces contrôles sont habituellement réalisés à la sortie de la chaîne de fabrication par des actions mécaniques, électriques ou optiques, effectuées à plusieurs postes successifs qui chacun contrôlent une ou plusieurs caractéristiques des spécifications du produit. Les contrôles mécaniques qui nécessitent un contact ne peuvent pas toujours être utilisés car ils peuvent être de nature à abîmer le produit fabriqué en fonction de son état ou de sa nature. C'est ainsi qu'en particulier des objets moulés ne peuvent pas être contrôlés à chaud, un contact mécanique risquant d'entraîner une déformation permanente.

Les contrôles optiques qui sont parfois utilisés, consistent à placer une ou plusieurs barrières photoélectriques sur le trajet des objets fabriqués. Chaque barrière photoélectrique est constituée par un pinceau lumineux unique, éventuellement modulé et dont l'occultation engendre un signal qui peut être utilisé pour fournir une information relative à la forme du produit. Ces mesures optiques nécessitent cependant l'utilisation de moyens de convoyage précis pour les produits car un léger déplacement du produit fabriqué au cours de son déplacement après fabrication, par exemple sur un tapis transporteur, entraîne une erreur importante dans la mesure. Le document GB-A-2 135 452 décrit un tel dispositif de détection optique dans lequel les bouteilles sont en outre entraînées en rotation dans le faisceau lumineux qui les éclaire

La fabrication automatique à haute cadence d'objets en verre ou en matière plastique moulés à haute température, par exemple dans des machines d'injection-soufflage, nécessite en particulier des contrôles nombreux et variés. Il en est ainsi notamment pour la fabrication des bouteilles pour lesquelles il est important d'éliminer toute bouteille qui présenterait certains défauts dangereux pour l'utilisateur ultérieur.

C'est ainsi que dans la fabrication des bouteilles en verre, par exemple au moyen de machines automatiques d'injection-soufflage multisections, on note de temps en temps l'apparition de défauts qui entraînent un rejet obligatoire des bouteilles correspondantes. Il s'agit par exemple de l'existence d'inclusions dans le matériau, formant des grains infondus, l'existence de filaments de verre entre les deux parois de la bouteille (défaut que l'on appelle communément "trapèze"), l'existence d'un excédent de matière sur la bague du goulot, etc...

D'autres défauts également importants nécessitent en général l'élimination des bouteilles qui les comportent. En particulier, le défaut de verticalité qui ne permet pas le remplissage dans les machines de remplissage automatique, l'apparition de microfissures ou un manque d'épaisseur dans certaines portions des parois, etc... Enfin, certains défauts secondaires tels que taches, plis, défauts de marquage, etc., doivent dans toute la mesure du possible être évités pour assurer une qualité constante des produits fabriqués.

Les machines de fabrication de ces objets et en particulier les machines automatiques d'injection-soufflage qui comportent une pluralité de sections sont commandées généralement par un automate programmable muni d'un microprocesseur et de systèmes à mémoires. La machine peut être pilotée en agissant sur les paramètres mémorisés dans le microprocesseur de façon à modifier les multiples caractéristiques de fonctionnement pour chacun des moules des différentes sections de la machine. Habituellement, ce pilotage est effectué par modification manuelle des données sur le clavier du microprocesseur qui commande la machine automatique.

Les contrôles de qualité nécessaires sont généralement effectués sur les produits après refroidissement. Comme on l'a vu précédemment en effet, il a toujours été considéré jusqu'à présent que les contrôles ne pouvaient pas être effectués dans des conditions correctes avant le refroidissement des produits. C'est ainsi par exemple que les bouteilles en verre, fabriquées dans des machines du type mentionné précédemment, présentent à leur sortie des moules une température considérée généralement comme trop importante pour permettre un contrôle correct. De plus, la matière des bouteilles est encore relativement peu résistante et risque de se briser avant d'avoir subi l'opération de recuit qui précède son refroidissement.

Les opérations de contrôle qui, dans cette application, doivent être faites sur l'intégralité des bouteilles fabriquées compte tenu des impératifs de la clientèle, se font donc sur les bouteilles après refroidissement, c'est-à-dire environ une heure après la fabrication. On comprendra que l'apparition de défauts systématiques ou l'augmentation de certains défauts particuliers ne permet pas en pratique d'agir immédiatement sur la machine de façon à effectuer une correction. De plus, il est extrêmement difficile de repérar avec certitude la provenance exacte des objets munis de défauts. Il n'est donc pas possible d'agir sur une section particulière de la machine de fabrication par l'intermédiaire du multiprocesseur de façon à éliminer avec certitude les défauts apparus.

Jusqu'à présent, on se contente donc le plus souvent de détruire les bouteilles qui comportent des défauts majeurs et d'agir manuellement de manière approximative sur la commande automatique de la machine de fabrication, ce qui ne permet d'éliminer qu'en partie les défauts, et après un temps de réaction important.

C'est ainsi que W. Sanders dans un article intitulé "Solid State Optics for Sidewall and Dimensional Inspection of Glassware" paru dans le N°3 de Juin 1981 de Glass Technology, s'il préconise bien de détecter un faisceau lumineux traversant des bouteilles en verre au moyen d'un réseau linéaire de diodes, non seulement ne prévoit pas de procéder à un contrôle de qualité sur les bouteilles à haute température, directement à la sortie d'une machine de moulage, mais encore se contente de la destruction des objets rejetés en cas de défaut ou en dehors de tolérances prédéterminées.

La demande de brevet EP-A1-0059 575 (EMHARDT) décrit un procédé de moulage de bouteilles en verre et a pour objet essentiel d'empêcher la poursuite de l'alimentation de la machine en gouttes de verre lorsqu'un incident est détecté dans la machine de formage qui lui fait immédiatement suite. Le procédé décrit comprend une première étape de formation de paraison à partir de gouttes de verre et une deuxième étape de formage d'une bouteille. Différents capteurs infrarouges sont placés à différents endroits de la machine et notamment dans un poste de recuit placé immédiatement en aval du poste de formation des paraisons. Un capteur de ce type est également placé à un poste où les objets formés se trouvent suspendus par un bras de transfert. Les contrôles effectués portent sur la fermeture correcte du moule, le nombre de paraisons, le nombre d'articles formés et la longueur des objets formés après recuit. De plus, les contrôles effectués sont du type tout ou rien et entraînent l'arrêt du fonctionnement de la machine en cas d'anomalie.

La présente invention a pour objet de réduire les défauts de qualité apparaissant lors de la fabrication d'objets en verre, notamment des bouteilles fabriquées au moyen de machines automatiques de moulage à haute cadence grâce à un contrôle de qualité automatique sans contact des objets.

L'invention a également pour objet un procédé dans lequel le résultat de la mesure effectuée sur les objets est utilisé pour modifier la fabrication immédiatement, afin d'éviter la reproduction des défauts constatés.

L'invention a encore pour objet de manière plus particulière le contrôle à chaud de produits moulés, en particulier de récipients ou bouteilles en matière plastique ou en verre, le contrôle s'effectuant sans contact, immédiatement à la sortie de la machine de fabrication.

Dans cette application particulière, l'invention a également pour objet de prévoir une rétroaction sur la fabrication en fournissant à l'automate programmable qui pilote la machine de moulage des informations résultant des mesures effectuées, de façon à modifier les caractéristiques de fonctionnement de la machine dans le but d'éviter la réapparition d'éventuels défauts constatés.

La présente invention a pour objet l'application de ce procédé général de contrôle automatique de qualité de bouteilles en verre à l'état chaud ainsi que le pilotage automatique de la machine de moulage en fonction du résultat de mesures effectuées.

Avec l'invention le contrôle à l'état chaud de la hauteur de bouteilles en verre moulé peut être exercé, ainsi que le pilotage automatique de la machine de fabrication pour éliminer les défauts constatés.

Selon l'invention, le procédé de contrôle automatique sans contact de la qualité d'objets moulés en matière plastique ou en particulier en verre à haute température fabriqués à haute cadence par une machine de moulage commandée automatiquement, consiste à faire se déplacer les objets sur un tapis transporteur devant un dispositif de capteur optique à la sortie de la machine de moulage, avant refroidissement sensible. On éclaire par un faisceau lumineux les objets en déplacement et on détecte la lumière transmise au moyen du capteur optique muni d'une barrette linéaire de mesure comportant une pluralité de photodiodes réceptrices, la barrette étant disposée avec son axe longitudinal orthogonal au déplacement des objets et au foyer d'un objectif de focalisation. On mémorise le résultat de la détection pour un nombre déterminé d'objets moulés et on agit périodiquement sur un ordinateur de commande de la machine de moulage en fonction des données mémorisées, en vue d'éliminer des défauts détectés dans la fabrication desdits objets moulés.

Dans un mode de réalisation préféré du procédé de l'invention, on régule en outre la puissance d'alimentation de la source d'éclairage en fonction du résultat de détection de façon à éviter la saturation des photodiodes de la barrette de mesure.

Dans un mode de réalisation préféré, l'initialisation de la mesure se fait en faisant se déplacer devant le dispositif de mesure optique un objet étalon dont on connaît les caractéristiques.

Toujours selon l'invention, le dispositif de contrôle sans contact en vue de la détection de défauts de la fabrication d'objets moulés en matière plastique et en particulier en verre à haute température fabriqués à haute cadence par une machine de moulage commandée automatiquement, les objets se déplaçant sur un tapis transporteur devant un capteur optique à la sortie de la machine de moulage et avant refroidissement sensible, comprend : un moyen d'éclairage des objets en déplacement devant le capteur optique qui est muni d'une barrette linéaire de mesure comportant une pluralité de photodiodes réceptrices, l'axe longitudinal de la barrette de mesure étant orthogonal au déplacement des objets; un objectif de focalisation dans le foyer duquel est disposée la barrette de mesure; un ordinateur de traitement recevant et mémorisant un nombre prédéterminé des signaux fournis par la barrette de mesure, et des moyens de connection entre l' ordinateur de traitement et la commande automatique de la machine de moulage de façon à pouvoir modifier la fabrication en fonction des mesures mémorisées en vue d' éliminer des défauts détectés dans la fabrication desdits objets moulés.

L'éclairage peut être fait au moyen d'une lumière quelconque. Dans un mode de réalisation préféré, on utilise une source de lumière linéaire dont l'axe longitudinal est parallèle à celui de la barrette de mesure. Une telle source est avantageusement constituée par un ou plusieurs tubes fluorescents alimentés en courant électrique dont l'intensité est régulée.

Pour éviter l'apparition de scintillations de la source lumineuse, le courant alimentant les tubes fluorescents est en outre de préférence un courant alternatif à haute fréquence et plus particulièrement à une fréquence telle que les tubes émettent de la lumière en continu. A cet effet la fréquence doit être supérieure à celle pour laquelle l'effet de rémanence de la décharge lumineuse dans le tube fluorescent assure une émission de lumière continue.

L'ordinateur de traitement comprend de préférence des moyens de mémorisation des signaux provenant des différentes photodiodes de l'appareil de mesure et une sortie de pilotage de la régulation de l'alimentation du moyen d'éclairage en fonction d'un seuil correspondant à la saturation des photodiodes.

Dans une réalisation pratique avantageuse du dispositif de l'invention, celui-ci comporte un ensemble d'émission-réception associé à la barrette de mesure, un comparateur recevant à la fois les signaux émis par les photodiodes de la barrette de mesure des signaux de seuil et un registre à décalage série/ parallèle associé à un convertisseur numérique/analogique relié au comparateur afin de lui fournir des signaux de seuil. Le comparateur émet alors un signal de sortie chaque fois que le signal émis par une photodiode dépasse le signal de seuil fourni.

Le capteur optique est de préférence protégé de l'environnement extérieur. Dans ce but, le dispositif comprend un corps de protection recevant l'appareil de mesure, l'objectif de focalisation et les organes de liaison électronique. L'une des extrémités du corps de protection constitue une chambre de dépoussiérage munie d'une paroi de fermeture comportant une fente ouverte parallèle à la barrette de mesure.

Un manchon entoure avantageusement le corps de protection en définissant un espace intercalaire de refroidissement qui communique avec la chambre de dépoussiérage qui se trouve ainsi mise en surpression.

L'invention sera mieux comprise à l'étude de quelques modes de réalisation décrits à titre d'exemple nullement limitatifs et illustrés par les dessins annexés, sur lesquels :

la figure 1 est une vue d'ensemble schématique du dispositif conforme à l'invention;

la figure 2 montre schématiquement les principaux éléments électroniques de traitement des signaux de mesure;

la figure 3 montre l'évolution dans le temps des différents signaux apparaissant dans certains points du circuit de la figure 2;

la figure 4 est une vue en coupe d'un exemple de réalisation du capteur optique utilisé dans le dispositif de l'invention, montrant notamment les moyens de protection contre l'environnement;

la figure 5 est une vue extérieure en bout de l'extrémité du corps de protection du capteur optique de la figure 4;

la figure 6 est une vue extérieure d'un objet étalon utilisé pour l'initialisation de la mesure;

Les exemples dans la description qui suit concernent une application du procédé général de l'invention au contrôle automatique de bouteilles en verre fabriquées par injection-soufflage dans une machine à commande automatique comportant une pluralité de sections individuelles chacune munie de deux moules. L'application comprend également l'action sur le pilotage de la machine de moulage en fonction du résultat des mesures effectuées.

On comprendra bien entendu que le procédé de l'invention puisse s'appliquer à toute autre fabrication d'objets en grandes quantités.

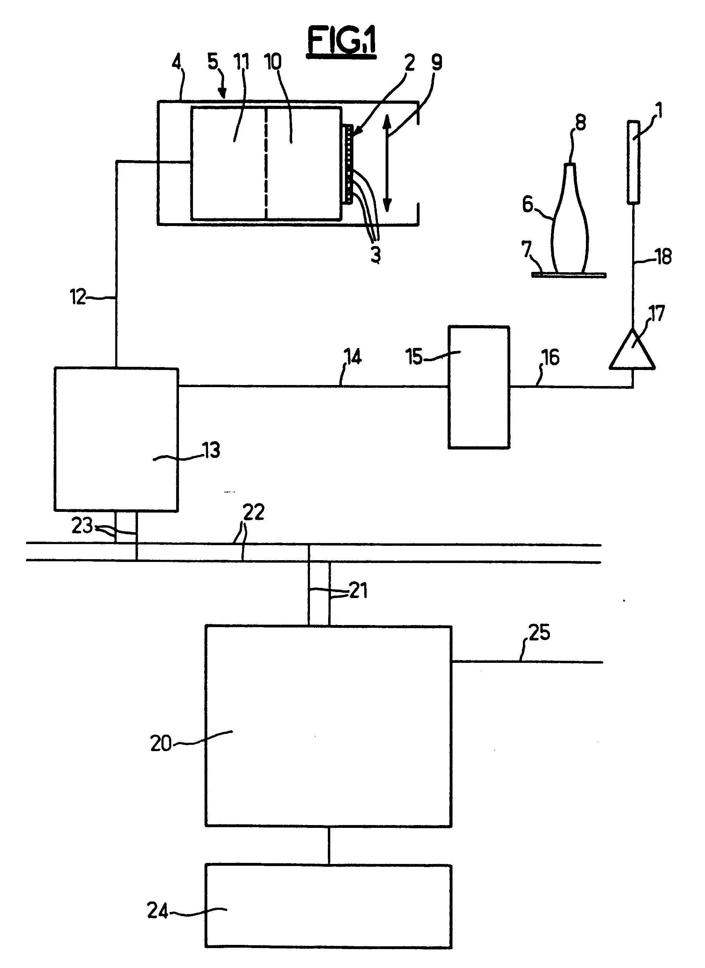

Tel qu'il est illustré sur la figure 1, le dispositif comprend un moyen d'éclairage en lumière blanche 1 qui peut être constitué avantageusement par un ou plusieurs tubes fluorescents, une barrette linéaire de mesure 2 munie d'une pluralité de photodiodes 3 et montée à l'intérieur d'un corps de protection 4, l'ensemble définissant un capteur optique 5.

Les bouteilles à contrôler, telles que la bouteille 6, se déplacent sur un tapis transporteur représenté schématiquement en 7 perpendiculairement au plan de la figure. L'axe longitudinal de la barrette de mesure 2 se trouve donc orthogonal au déplacement des bouteilles 6. On notera que dans l'exemple de réalisation, la source de lumière 1 est avantageusement constituée par un tube fluorescent dont l'axe longitudinal est parallèle à celui de la barrette de mesure 2. Un capot de protection non illustré sur la figure peut avantageusement être prévu pour entamer la source lumineuse 1 et la maintenir sous pression d'air comprimé afin d'éviter la pénétration de poussières.

Dans la disposition illustrée sur la figure 1, la partie supérieure du goulot 8 de la bouteille 6 se trouve sensiblement alignée avec le centre de la barrette de mesure 2. En effet, la figure 1 illustre à titre d'exemple une application du procédé de l'invention à la mesure de hauteur des bouteilles 8 de façon à détecter un éventuel défaut de hauteur. Bien entendu, dans le cas où le procédé est utilisé pour détecter un autre type de défaut, le capteur optique 5 et la source lumineuse 1 seront placés d'une manière différente de façon à apprécier au mieux le défaut considéré.

L'essentiel pour l'application du procédé de l'invention est que l'axe longitudinal de la barrette de mesure 2 soit orthogonal au déplacement des bouteilles 8 de façon que la combinaison de la disposition linéaire des photodiodes 3 avec le déplacement orthogonal des bouteilles 8 permette d'obtenir, par le moyen simple que constitue la barrette de mesure 2, une analyse et une reconnaissance de forme par traitement numérique de l'image fournies par les photodiodes en fonction du déplacement des bouteilles.

La barrette de mesure 2 est en outre disposée au foyer d'un objectif de focalisation 9.

Les signaux émis par les photodiodes 3 sont amplifiés ou traités par le bloc d'amplification 10. Le capteur 5 comporte en outre un ensemble d'émission-réception 11 connectées par la connexion 12 à l'interface 13 qui agit par la connexion 14 sur la commande d'alimentation et de puissance 15 de la source de lumière 1. A cet effet, la commande d'alimentation est reliée par la connexion 16 à l'amplificateur 17 lui-même connecté par la connexion 18 à la lampe 1.

Un ordinateur de traitement 20 est relié par les connexions 21,22 et 23 a l'interface 13. Le dialogue avec l'ordinateur de traitement 20 est possible par l'intermédiaire du clavier 24. Par ailleurs, l'ordinateur de traitement 20 est capable d'envoyer des signaux de commande à l'automate programmable qui pilote la machine d'injection-soufflage des bouteilles 8 par l'intermédiaire de la connexion 25.

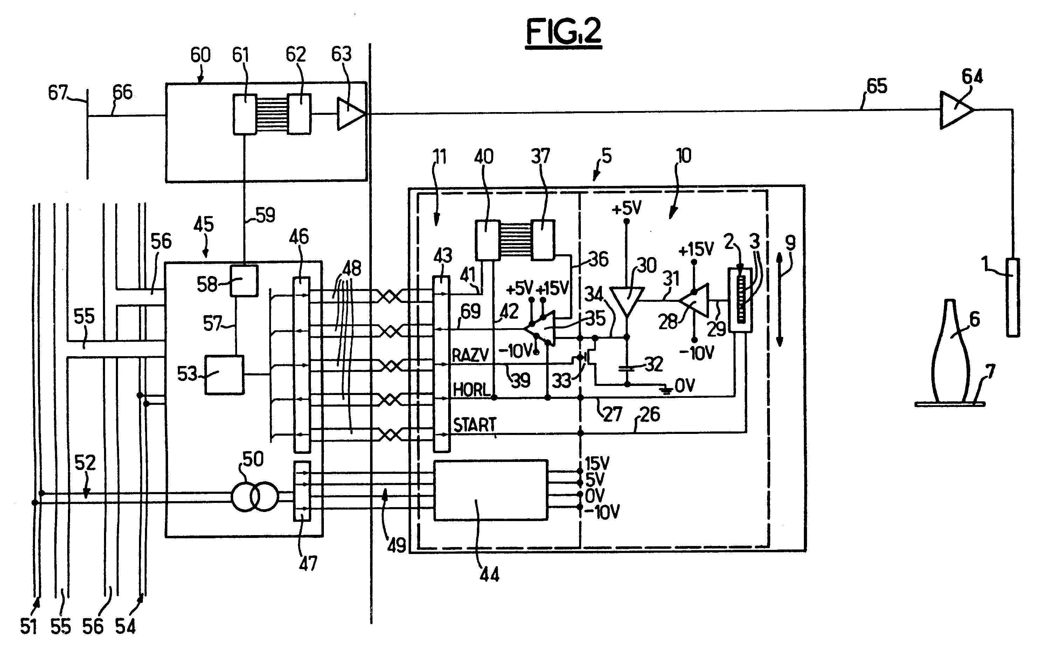

En se référant à la figure 2, qui montre l'essentiel des éléments électroniques du dispositif de la figure 1, on retrouve les éléments analogues portant les mêmes références.

Le bloc d'amplification analogique 10 reçoit les signaux de la barrette de mesure 2 placés comme il a été dit dans la plan focal de l'objectif 9. Le réseau est alimenté par deux tensions continues -10 et +5 volts et deux signaux numériques : le signal start véhiculé par la connexion 26 et le signal horloge véhiculé par la connexion 27. Le réseau délivre des trains d'impulsions synchrones avec les signaux start et horloge amplifiés par un amplificateur opérationnel rapide 28 relié par la connexion 29 à la barrette de mesure 2. Le signal issu de l'amplificateur 28 est intégré dans un circuit d'intégration comprenant un convertisseur tension-courant 30 relié par la connexion 31 à l'amplificateur 28 et un condensateur d'intégration 32 aux bornes duquel est monté un commutateur statique rapide 33.

Le signal apparaissant à la sortie du condensateur 32 sur la connexion 34 alimente un comparateur rapide 35 par l'une de ses entrées. Le seuil de ce comparateur est fixé par la sortie 36 d'un convertisseur numérique/analogique 37. Après chaque impulsion apparaissant à l'entrée du condensateur 32, celui-ci est déchargé par le commutateur statique 33 alimenté par un signal de remise à zéro RAZV véhiculé par la connexion 39.

Le seuil analogique du convertisseur numérique/analogique 37 est fixé par la valeur numérique à l'entrée elle-même définie par un transfert-série à l'entrée du registre à décalage 40 à' entrée-série et sortie parallèle connecté au réseau par la liaison 41 et recevant le signal d'horloge par la connexion 42.

Les différents signaux à l'émission et à la réception transitent par un module 43 d'émission/réception des lignes.

L'ensemble est encore complété par un dispositif régulateur de tension 44.

Le capteur 5 est associé à une carte ordinateur 45 possédant un système d'émission-réception de lignes 46 connecté à l'émetteur-récepteur 43 ainsi qu'un dispositif de prérégulation des tensions 47 connecté au régulateur 44.

Le capteur 5 est relié à la carte ordinateur par les câbles 48 et 49 qui comprennent des paires torsadées pour les signaux électriques (câbles 48) et des fils simples pour les alimentations (câbles 49).

Le prérégulateur de tension 47 est alimenté en 400 Hz à l'aide d'un tranformateur torique 50 non rayonnant branché sur la ligne d'alimentation générale à 24 volts 400 Hz référencé 51 par l'intermédiaire de la connexion 52.

La carte ordinateur possède une unité centrale 53 avec une mémoire vive importance et une mémoire morte comme illustré sur la figure.

La carte ordinateur est branchée sur une ligne d'alimentation à 5 volts référencée 54 pour l'alimentation des circuits intégrés. La carte ordinateur 45 est également connectée à un bus de décision locale 55 et à un bus général de transfert de données 56.

Les signaux fournis par l'unité centrale 53 sont transmis par la connexion 57 à un bloc de mise en forme 58 puis par la connexion 59 à une carte de puissance d'éclairage référencée 60 dans son ensemble. Cette dernière comprend un registre à décalage série/parallèle référencé 61 connecté à un convertisseur numérique-analogique 62. Le signal de sortie amplifié par l'amplificateur de puissance 63 est alors transmis par l'intermédiaire d'un convertisseur tension-fréquence 64 relié par la connexion 65 à la source d'éclairage 1. La carte puissance d'éclairage 60 est reliée par la connexion 66 à la ligne de puissance d'élairage 67 à 30 volts.

La variation de tension à l'entrée du convertisseur 64 provoque des variations d'intensité du tube fluorescent 1. Il est ainsi possible d'obtenir une puissance régulée afin d'éviter une éventuelle saturation nuisible des photodiodes.

Le convertisseur 64 alimente le tube 1 en courant à haute fréquence par exemple de l'ordre de 25 000 Hz afin d'utiliser la rémanence de la décharge lumineuse dans le tube 1 pour obtenir une émission de lumière continue c'est-à-dire sans scintillation.

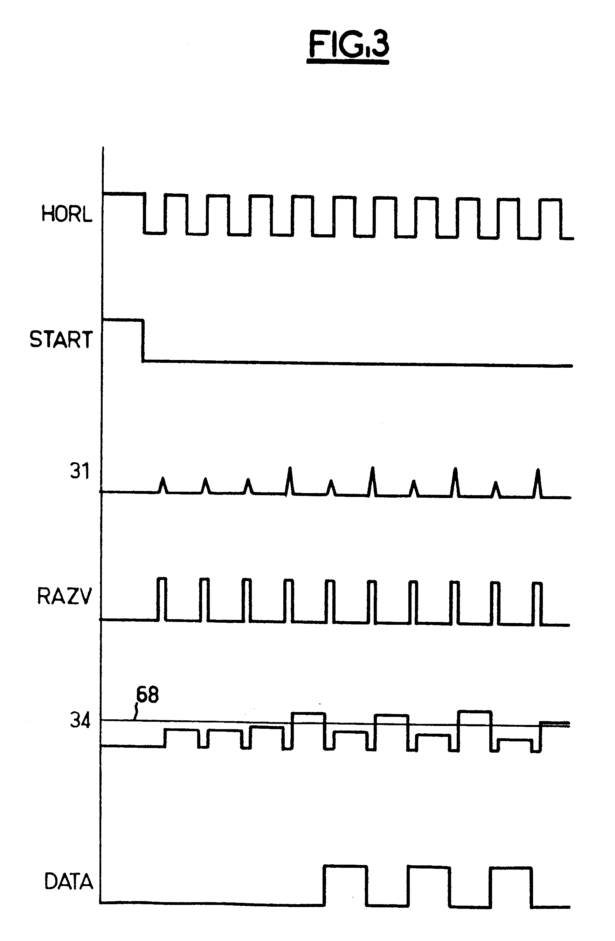

En se référant à la figure 3, on voit les différents signaux de fonctionnement du capteur 5. Le premier signal en haut de la figure est le signal d'horloge qui fait fonctionner le registre à décalage 40 du réseau de photodiodes 2, avec le signal start qui est le deuxième en partant du haut sur la figure 3.

Le signal apparaissant à la connexion 31 à la sortie de l'amplificateur 28 n'est pas exploitable directement. Le signal RAZV appliqué sur la grille du commutateur 33 fournit le signal apparaissant sur la connexion 34. Ce signal pénètre dans le comparateur 35 où il est comparé à la tension de seuil 36 (droite 68 sur la figure 3), de façon à fournir les signaux référencés data issus du comparateur 35 véhiculés par la connexion 69. Ce sont ces signaux numériques qui sont alors transférés sur la carte ordinateur 45.

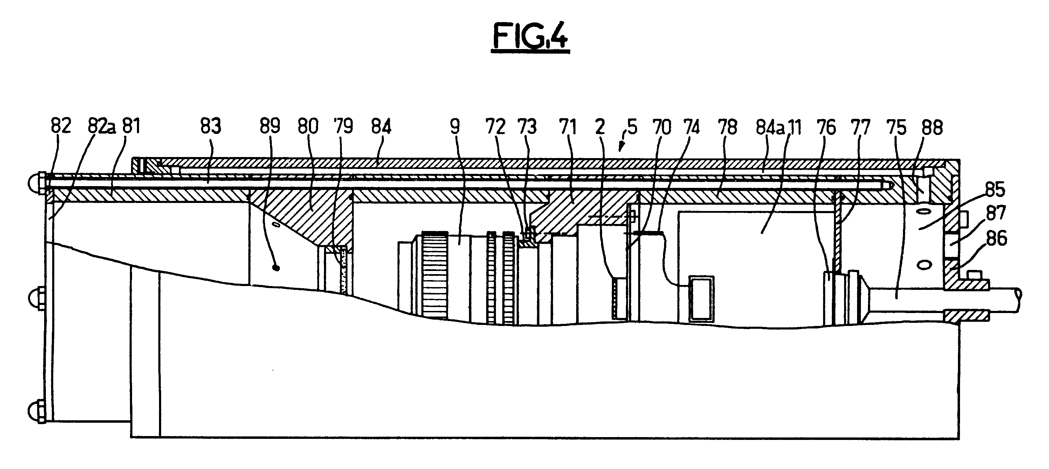

La figure 4 montre un mode de réalisation pratique du capteur 5. Ou retrouve sur la figure 4 l'objectif de focalisation 9 et la barrette de photodiodes 2 disposés dans le plan focal de l'objectif 9. La barrette 2 est directement fixée sur la plaque 70 vissée dans le porte objectif 71. Le circuit de traitement et d'amplification analogique est monté sur la plaque 70. L'objectif 9 est fixé sur le porte objectif 71 par l'intermédiaire d'une bague d'adaptation 72 et au moyen de vis 73. Les signaux amplifiés sont transmis par la connexion 74 à la carte d'émission-réception 11. Les signaux après transformation sont transmis par le câble de liaison électronique 75 dont l'embout 76 est solidaire d'une plaque radiale 77 montée sur le corps 78.

A l'avant de l'objectif 9 se trouve monté un filtre anticalorique 79 disposé dans un bloc porte-filtres 80. La barrette de photodiodes 3 est en effet très sensible au proche infra-rouge. Si le contrôle est fait sur des objets rayonnants dans la zone de longueur d'ondes proche du micron, tels que par exemple des bouteilles en verre en sortie de moule, il convient d'utiliser un tel filtre afin que ce rayonnement ne vienne pas perturber la réception du rayonnement lumineux émis dans le visible par la source 1.

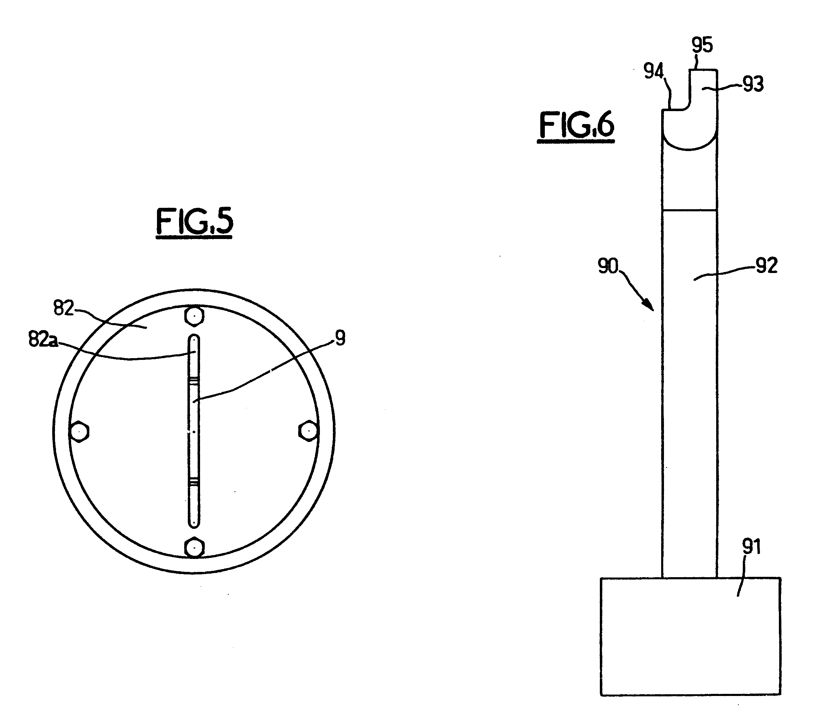

Une chambre de dé poussiérage 81 à paroi cylindrique est montée à l'extrémité avant du capteur 5. Elle comporte, comme on peut le voir sur la figure 5, une paroi frontale d'obturation 82 munie d'une fente ouverte 82a disposée selon un diamètre.

L'ensemble des éléments qui constituent le capteur 5 est solidarisé par des vis de serrage axial 83. Un manchon cylindrique 84 entoure le corps du capteur 5 en laissant subsister un intervalle 84a.

Une chambre d'alimentation en air sous pression référencée 85 se trouve à la partie arrière du capteur 5 et est délimitée à l'arrière par une cloison frontale 86 munie d'un orifice d'entrée d'air sous pression 87. L'air alimenté par l'orifice 87 pénètre par les trous radiaux 88 dans l'intervalle annulaire 84a et s'écoule dans cet intervalle produisant ainsi un refroidissement du capteur. Des trous 89 percés dans le porte-filtre 80 débouchent dans l'intervalle 84a et dans la chambre de dépoussiérage 81 en mettant celle-ci en surpression. De cette manière, on évite que des poussières extérieures ne puissent gêner le fonctionnement du capteur.

Pour permettre l'initialisation de la mesure et le début de fonctionnement du système, on utilise un objet étalon illustré sur la figure 6 et référencé 90 dans son ensemble. Dans l'application décrite ici à titre d'exemple concernant des bouteilles, la hauteur totale de l'étalon 90 correspond sensiblement à la hauteur d'une bouteille. De manière plus précise, on voit que l'étalon 90 comporte un socle 91 et une tige 92 au sommet de laquelle se trouve une saillie 93 sur la moitié de l'épaisseur de la tige 92. On définit ainsi un niveau bas 94 et un niveau haut 95. La différence de niveaux peut être par exemple de l'odre de 20 mm. La hauteur moyenne d'une bouteille à haute température telle qu'elle sort de la machine de fabrication est située à égale distance des niveaux 94 et 95.

Le dispositif fonctionne alors de la manière suivante, en se référant aux figures 1 et 6. On place sur le tapis transporteur 1 l'objet étalon 90 de façon que la partie supérieure en saillie 96 munie des deux surfaces 94 et 95 se présente de face entre la barrette de mesure 2 et la source lumineuse 1. Au cours de son déplacement devant le capteur 5, la partie en saillie 93 masque donc par sa partie pleine se trouvant au-dessous du niveau 94 un certain nombre de photodiodes de la barrette de mesure 2. Lorsque le déplacement se poursuit, un plus grand nombre de photodiodes se trouve masqué par la partie pleine se trouvant au-dessous du niveau 95.

La succession de ces deux signaux provoquée par le déplacement d'un objet étalon 90 peut alors être reconnue par l'ordinateur de traitement et en particulier l'unité centrale 53 (figure 2).

Dans ces conditions, au moment du passage de l'étalon 90, l'ordinateur note le temps Te. Connaissant ensuite les intervalles de temps qui s'écoulent entre la fabrication de chaque bouteille, l'ordinateur peut eu déduire eu tenant compte des informations relatives à la position initiale de l'étalon, la provenance exacte de chaque bouteille mesurée. En d'autres termes, l'ordinateur est capable de repérer quelle est la section de la machine de moulage qui a fabriqué une bouteille déterminée sur laquelle un défaut est détecté. Il en résulte que l'ordinateur est également capable de fournir un signal de commande à l'automate de pilotage de la machine afin de modifier une caractéristique de fonctionnement en fonction du défaut détecté pour une section déterminée de la machine de moulage.

Dans une application consistant à vérifier la hauteur des bouteilles fabriquées, la mesure se fait en déterminant au passage de chaque bouteille le nombre de diodes occultées. Au moment de la mesure, on indique également et on mémorise dans l'ordinateur de traitement le moment où la mesure est faite. La mesure de la hauteur réelle est calculée par l'ordinateur en fonction du nombre de photodiodes occultées et connaissant la hauteur exacte des deux niveaux 94 et 95 de la bouteille étalon 90. Le repérage des sections de la machine de moulage se fait par le calcul de l'intervalle de temps écoulé au moment du passage d'une bouteille déterminée.

De façon que les bouteilles ne présentent pas de transparence, ce qui pourrait fausser la mesure, il est convenable qu'avec un objet opaque, on effectue une opération de régulation de lumière afin de compenser les dérives de sensibilité du capteur ainsi que les dérives de luminosité de l'éclairage. Dans ce but, après le passage de chaque bouteille et après avoir noté le moment de passage, la fréquence de mesure du capteur 5 est doublée. Dans ces conditions, sa sensibilité lumineuse se trouve divisée par deux. A cet instant et en l'absence de toute bouteille devant le champ de vision, l'unité centrale 53 regarde si le niveau du signal apparaissant sur la connexion 34 à l'entrée du comparateur 35 (figure 2) est supérieur ou inférieur au seuil choisi. Dans le cas où le niveau est inférieur au seuil choisi, l'unité centrale transmet au travers de la carte puissance 60 une unité de tension supplémentaire, ce qui augmente la luminosité de la source 1. Dans le cas où le niveau est supérieur, l'unité centrale transmet au contraire un signal tendant à diminuer l'intensité de l'éclairage de la source 1.

Le procédé de l'invention permet ainsi non seulement un contrôle de qualité fait à chaud immédiatement à la sortie de la machine de fabrication, mais également une régulation automatique par rétroaction sur le fonctionnement même de la machine de fabrication.

Le procédé de l'invention réalise une approximation d'une reconnaissance de forme grâce à l'utilisation d'une pluralité de photodiodes disposées selon une barrette linéaire orthogonalement au déplacement des objets à contrôler.

L'utilisation d'un éclairage en lumière quelconque permet d'assurer l'éclairage complet de l'objet quelle que soit sa forme et ainsi d'obtenir une mesure précise par occultation d'un certain nombre de photodiodes en fonction du déplacement de l'objet.

Grâce à l'invention, il devient possible d'effectuer, à chaud, une pluralité de contrôles sur les bouteilles. On peut en particulier par une analyse de la forme générale de chaque bouteille détecter des bouteilles collées les unes aux autres ou couchées sur le tapis de transport. On peut également détecter par une analyse de la forme des bouteilles, les bouteilles trop petites ou inclinées sur la verticale. Dans tous les cas on peut commander l'éjection des bouteilles défactueuses et, si l'incident se répète pour une section déterminée de la machine de fabrication, rétroagir directement sur la machine de fabrication afin d'éliminer cet incident. Il est également possible, grâce à l'invention, de compter les bouteilles fabriquées par chaque section et de mesurer directement la vitesse réelle du tapis transporteur.

**Revendications**

1. Procédé de contrôle automatique sans contact de la qualité d'objets moulés (6) en matière plastique ou en particulier en verre à haute température fabriqués à haute cadence par une machine de moulage commandée automatiquement, procédé dans lequel: les objets se déplacent sur un tapis transporteur (7) devant un dispositif de capteur optique (5) à la sortie de la machine de moulage, avant refroidissement sensible; on éclaire par un faisceau lumineux (1) les objets en déplacement; on détecte la lumière transmise au moyen du capteur optique (5) muni d'une barrette linéaire de mesure (2) comportant une pluralité de photodiodes réceptrices (3), la barrette (2) étant disposée avec son axe longitudinal orthogonal au déplacement des objets (6) et au foyer d'un objectif de focalisation (9); on mémorise le résultat de la détection pour un nombre déterminé d'objets moulés; et on agit périodiquement sur un ordinateur de commande de la machine de moulage en fonction des données mémorisées, en vue d'éliminer des défauts détectés dans la fabrication desdits objets moulés.

2. Procédé selon la revendication 1, caractérisé par le fait que l'on régule la puissance d'alimentation de la source d'éclairage (1) en fonction du résultat de la détection de façon à éviter la saturation des photodiodes.

3. Procédé selon l'une quelconque des revendications précédentes, caractérisé par le fait qu'après le passage de chaque objet, on augmente la fréquence de mesure du capteur, diminuant ainsi sa sensibilité lumineuse, on compare alors le signal reçu à un seuil déterminé et on augmente la puissance de la source lumineuse si le niveau est inférieur au seuil déterminé, et on diminue l'intensité de la source lumineuse si le niveau est supérieur au seuil déterminé.

4. Procédé de contrôle automatique selon l'une quelconque des revendications précédentes, caractérisé par le fait que l'initialisation de la mesure se fait en faisant se déplacer devant le dispositif de mesure (5) un objet étalon (90) dont on connaît les caractéristiques.

5. Dispositif de contrôle sans contact en vue de la détection de défauts de la fabrication d'objets moulés en matière plastique ou en particulier en verre à haute température fabriqués à haute cadence par une machine de moulage commandée automatiquement, les objets se déplaçant sur un tapis transporteur devant un capteur optique à la sortie de la machine de moulage et avant refroidissement sensible, le dispositif comprenant : un moyen d'éclairage des objets en déplacement devant le capteur optique, qui est muni d'une barrette linéaire de mesure (2) comportant une pluralité de photodiodes réceptrices (3), l'axe longitudinal de la barrette de mesure étant orthogonal au déplacement des objets (6); un objectif de focalisation (9) dans le foyer duquel est disposée la barrette de mesure (2); un ordinateur de traitement (20) recevant et mémorisant un nombre prédéterminé des signaux fournis par la barrette de mesure, et des moyens de connection (25) entre l'ordinateur de traitement (20) et la commande automatique de la machine de moulage de façon à pouvoir modifier la fabrication en fonction des mesures mémorisées en vue d'éliminer des défauts détectés dans la fabrication desdits objets moulés.

6. Dispositif de contrôle selon la revendication 5, dans lequel le moyen d'éclairage est un tube fluorescent dont l'axe longitudinal est parallèle à celui de la barrette de mesure, ledit moyen d'éclairage étant alimenté en courant électrique d'intensité régulée et de fréquence supérieure à la rémanence du tube fluorescent.

7. Dispositif selon l'une des revendications 5 ou 6, caractérisé par le fait que l'ordinateur de traitement (20) comprend des moyens de memorisation des signaux provenant des différentes photodiodes (3) de la barrette de mesure (2) et une sortie de pilotage (14) de la régulation de l'alimentation du moyen d'éclairage (1) en fonction d'un seuil correspondant à la saturation des photodiodes.

8. Dispositif selon l'une des revendications 5 à 7, caractérisé par le fait qu'il comprend un ensemble d'émission-réception (11) associé à la barrette de mesure (2), lequel comprend un comparateur (35) recevant à la fois la succession des signaux émis par les photodiodes (3) de la barrette de mesure (2) et une succession de signaux de seuil (36), et un registre à décalage (40) série/parallèle associé à un convertisseur numérique/analogique (37) relié au comparateur (35) afin de lui fournir les signaux de seuil précités, le comparateur (35) émettant un signal de sortie chaque fois que le signal émis par une photodiode (3) dépasse le signal de seuil fourni.

9. Dispositif selon la revendication 8, caractérisé par le fait que l'ensemble d'émission-réception comprend en outre un commutateur (33) de remise à zéro du signal d'entrée du comparateur (35).

10. Dispositif selon l'une quelconque des revendications 5 à 9, caractérisé par le fait qu'il comprend un corps de protection (71) recevant la barrette de mesure (2), l'objectif de focalisation (9) et les circuits d'électronique, une des extrémités dudit corps constituant une chambre de dépoussiérage (81) munie d'une paroi de fermeture (82) comportant une fente ouverte (82e) disposée parallèlement à la barrette de mesure (2).

11. Dispositif selon la revendication 10, caractérisé par le fait qu'un filtre de protection anticalorique (79) est monté entre la chambre de dépoussiérage (81) et l'objectif (9).

12. Dispositif selon les revendications 10 ou 11, caractérisé par le fait qu'un manchon (84) entoure le corps de protection en définissant un espace intercalaire (84a) de refroidissement à l'intérieur duquel peut circuler un écoulement d'air sous pression, ledit espace communiquant avec la chambre de dépoussiérage (81) qui se trouve ainsi mise en surpression.

**Claims**

1. Process for the automatic, contactless monitoring of the quality of objects (6) moulded in a plastics material or in particular in glass at a high temperature and a rapid rate by an automatically controlled moulding machine, in which process: the objects move on a conveyor belt (7) in front of an optical sensor device (5) at the outlet of the moulding machine, before appreciable cooling occurs; the objects are illuminated by a beam of light (1) while moving; the detection of the light emitted takes place by means of the optical sensor (5), which is provided with a straight measuring rod (2) comprising a plurality of receiving photodiodes (3), the rod (3) being arranged with its longitudinal axis orthogonal to the movement of the objects (6) and to the focal point of a focusing lens (9); the result of the detection is stored for a predetermined number of moulded objects; and a computer for controlling the moulding machine in dependence upon the stored data is acted upon periodically, for the purpose of eliminating faults detected in the production of the said moulded objects.

2. Process according to claim 1, characterised in that the power for supplying the lighting source (1) is adjusted in dependence upon the result of the detection, in order to prevent the saturation of the photodiodes.

3. Process according to any one of the preceding claims, characterised in that, after each object has passed, the measuring frequency of the sensor is increased, thus decreasing the sensitivity to light, the signal received is then compared with a predetermined threshold and the power of the light source is increased if the level is below the predetermined threshold, and the intensity of the light source is decreased if the level is above the predetermined threshold.

4. Process for automatic monitoring according to any one of the preceding claims, characterised in that the initialisation of measurement is carried out by moving a standard object (90) having known characteristics in front of the measuring device (5).

5. Contactless monitoring device for the purpose of detecting faults in the production of objects (1) moulded in a plastics material or in particular in glass at a high temperature and produced at a rapid rate by an automatically controlled moulding machine, the objects moving on a conveyor belt in front of an optical sensor at the outlet of the moulding machine and before appreciable cooling occurs, the device comprising: a lighting means for the objects moving in front of the optical sensor, which sensor is provided with a straight measuring rod (2) comprising a plurality of receiving photodiodes (3), the longitudinal axis of the measuring rod being orthogonal to the movement of the objects (6); a focusing lens (9), at the focal point of which there is arranged a measuring rod (2); a processing computer (20) receiving and storing a predetermined number of the signals supplied by the measuring rod, and connection means (25) between the processing computer (20) and the automatic control of the moulding machine, so that production can be modified in dependence upon the stored measurements, for the purpose of eliminating faults detected in the production of the said moulded objects.

6. Monitoring device according to claim 5, in which the lighting means is a fluorescent tube, the longitudinal axis of which is parallel to that of the measuring rod, the said lighting means being supplied with electrical current at a regulated intensity and at a frequency greater than the remanence of the fluorescent tube.

7. Device according to one of claims 5 or 6, characterised in that the processing computer (20) comprises means for storing the signals coming from the various photodiodes (3) of the measuring rod (2) and a control output (14) for the regulation of the supply to the lighting means (1) in dependence upon a threshold corresponding to the saturation of the photodiodes.

8. Device according to one of claims 5 to 7, characterised in that it comprises a transmitting-receiving assembly (11) associated with the measuring rod (2), which assembly comprises a comparator (35) receiving both the series of signals transmitted by the photodiodes (3) of the measuring rod (2) and a series of threshold signals (36), and a series/parallel shift register (40) associated with a digital-to-analogue converter (37) connected to the comparator (35) in order to supply it with the above-mentioned threshold signals, the comparator (35) transmitting an output signal each time the signal transmitted by a photodiode (3) exceeds the threshold signal supplied.

9. Device according to claim 8, characterised in that the transmitting-receiving assembly further comprises a reset switch (33) for the input signal of the comparator (35).

10. Device according to any one of claims 5 to 9, characterised in that it comprises a protective body (71) accommodating the measuring rod (2), the focusing lens (9) and the electronics circuits, one of the ends of the said body forming a dedusting chamber (81) provided with a closing wall (82) comprising an open slit (82a) arranged parallel to the measuring rod (2).

11. Device according to claim 10, characterised in that a heat-resistant protective filter (79) is mounted between the dedusting chamber (81) and the lens (9).

12. Device according to claims 10 or 11, characterised in that a sleeve (84) surrounds the protective filter, defining an interposed cooling space (84a) inside which a pressurised air flow can circulate, the said space communicating with the dedusting chamber (81), which is thus placed under a high pressure.

**Patentansprüche**

1. Verfahren zur kontaktlosen automatischen Qualitätskontrolle von mittels einer automatisch gesteuerten Formungsmaschine in hoher Geschwindigkeit hergestellten, aus Kunststoffmaterial oder insbesondere aus Glas hoher Temperatur geformten Gegenständen, wobei in dem Verfahren: sich die Gegenstände vor einer merklichen Abkühlung auf einem Transportband (7) an einer am Ausgang der Formungsmaschine befindlichen optischen Erfassungseinrichtung (5) vorbei verschieben; man die Gegenstände bei ihrer Verschiebung mittels eines Strahlenbündels (1) beleuchtet; man das transmittierte Licht mittels der optischen Erfassungseinrichtung (5) erfaßt, die mit einer eine Anzahl von Empfangsphotodioden (3) umfassenden linearen stabförmigen Meßanordnung (2) versehen ist, wobei die stabförmige Anordnung (2) mit ihrer longitudinalen Achse orthogonal zur Verschiebungsrichtung der Gegenstände (6) und im Brennpunkt eines Abbildungsobjektivs (9) angeordnet ist; man das Ergebnis der Erfassung für eine bestimmte Anzahl von geformten Gegenständen abspeichert; und man in Abhängigkeit von den abgespeicherten Daten periodisch auf eine Prozessteuereinrichtung der Formungsmaschine einwirkt, um die erfaßten Fehler bei der Herstellung der geformten Gegenstände zu eliminieren.

2. Verfahren nach Anspruch 1 **dadurch gekennzeichnet**, daß man die Versorgungsleistung der Lichtquelle (1) in Abhängigkeit vom Ergebnis der Erfassung derart regelt, daß eine Sättigung der Photodioden vermieden wird.

3. Verfahren nach einem der vorhergehenden Ansprüche, **dadurch gekennzeichnet**, daß man nach dem Durchgang jedes Gegenstandes die Meßfrequenz der Erfassungseinrichtung unter Verminderung der Lichtempfindlichkeit erhöht, man dann das empfangene Signal mit einer vorgegebenen Schwelle vergleicht und die Leistung der Lichtquelle erhöht, wenn das Niveau geringer als die vorgegebene Schwelle ist, und man die Intensität der Lichtquelle vermindert, wenn das Niveau die vorgegebene Schwelle übersteigt.

4. Automatisches Kontrollverfahren nach einem der vorhergehenden Ansprüche, **dadurch gekennzeichnet**, daß die Initialisierung der Messung erfolgt, indem man die Verschiebung eines Standardgegenstandes (90) mit bekannten Eigenschaften an der Meßvorrichtung (5) vorbei bewirkt.

5. Vorrichtung zur kontaktlosen Kontrolle zur Erfassung von Fehlern bei der Herstellung von mittels einer automatisch gesteuerten Formungsmaschine in hoher Geschwindigkeit hergestellten, aus Kunststoffmaterial oder insbesondere aus Glas hoher Temperatur geformten Gegenständen, bei der sich die Gegenstände vor einer merklichen Abkühlung auf einem Transportband an einer am Ausgang der Formungsmaschine befindlichen optischen Erfassungseinrichtung vorbei verschieben, wobei die Vorrichtung enthält: eine Einrichtung zur Beleuchtung der Gegenstände bei ihrer Verschiebung vorbei an der optischen Erfassungseinrichtung, welche mit einer eine Anzahl von Empfangsphotodioden (3) umfassenden, mit ihrer longitudinalen Achse orthogonal zur Verschiebungsrichtung der Gegenstände (6) angeordneten linearen stabförmigen Meßanordnung (2) versehen ist; ein Abbildungsobjektiv (9), in dessen Brennpunkt die stabförmige Meßanordnung (2) angeordnet ist; eine Datenverarbeitungseinrichtung (20) zur Aufnahme und Abspeicherung einer vorgegebenen Anzahl von durch die stabförmige Meßanordnung gelieferten Signalen; und Mittel zur Verbindung (25) zwischen der Datenverarbeitungseinrichtung (20) und der automatischen Steuerung der Formungsmaschine in der Weise, daß die Herstellung in Abhängigkeit von den abgespeicherten Messungen modifiziert werden kann, um die erfaßten Fehler bei der Herstellung der geformten Gegenstände zu eliminieren.

6. Vorrichtung nach Anspruch 5, bei der die Beleuchtungseinrichtung eine Fluoreszenzröhre ist, deren longitudinale Achse sich parallel zu der der stabförmigen Meßanordnung befindet, wobei die Beleuchtungseinrichtung mit elektrischem Strom geregelter Stärke und einer die Remanenz der Fluoreszenzröhre übersteigenden Frequenz gespeist wird.

7. Vorrichtung nach einem der Ansprüche 5 oder 6, **dadurch gekennzeichnet**, daß die Datenverarbeitungseinrichtung (20) Mittel zum Abspeichern der von den verschiedenen Photodioden (3) der stabförmigen Meßanordnung (2) herstammenden Signale und einen Steuerausgang (14) zur Regulierung der Versorgung der Beleuchtungseinrichtung (1) in Abhängigkeit von einer der Sättigung der Photodioden entsprechenden Schwelle umfaßt.

8. Vorrichtung nach einem der Ansprüche 5 bis 7, **gekennzeichnet durch** eine der stabförmigen Meßanordnung (2) zugeordnete Sender-Empfänger-Baugruppe (11), die einen die Folge der von den Photodioden (3) der stabförmigen Meßanordnung (2) abgegebenen Signale und eine Folge von Schwellwertsignalen (36) gleichzeitig aufnehmenden Vergleicher (35) und ein einem mit dem Vergleicher (35) zur Zuführung der oben erwähnten Schwellwertsignale zu diesem gekoppelten Digital/Analog-Wandler (37) zugeordnetes serielles/paralleles Schieberegister (40) umfaßt, wobei der Vergleicher (35) jedesmal ein Ausgangssignal abgibt, wenn das durch eine Photodiode (3) abgegebene Signal das gelieferte Schwellwertsignal überschreitet.

9. Vorrichtung nach Anspruch 8, **dadurch gekennzeichnet**, daß die Sender-Empfänger-Baugruppe weiterhin eine Schalteinrichtung (33) zum Zurücksetzen des Eingangssignals des Vergleichers (35) auf Null enthält.

10. Vorrichtung nach einem der Ansprüche 5 bis 9, **dadurch gekennzeichnet**, daß sie einen die stabförmige Meßanordnung (2), das Abbildungsobjectiv (9) und die elektronischen Schaltungen aufnehmenden Schutzkörper (71) aufweist, wobei ein Ende des Körpers eine Staubschutzkammer (81) bildet, die mit einer einen parallel zu der stabförmigen Meßanordnung (2) angeordneten offenen Schlitz (82a) aufweisenden Verschlußwand (82) versehen ist.

11. Vorrichtung nach Anspruch 10, **dadurch gekennzeichnet**, daß zwischen der Staubschutzkammer (81) und dem Objektiv (9) ein Wärmeschutzfilter (79) angebracht ist.

12. Vorrichtung nach den Ansprüchen 10 oder 11, **dadurch gekennzeichnet,** daß der Schutzkörper von einer einen dazwischen befindlichen Kühlzwischenraum, in dessen Innerem ein Strom von Druckluft zirkulieren kann, begrenzenden Hülse (84) umgeben ist, wobei der Zwischenraum mit der Staubschutzkammer (81) kommuniziert, die somit ebenfalls unter Überdruck gesetzt ist.

# FIG.1

## FIG.2

EP 0 177 004 B1

EP 0 177 004 B1

# FIG.3

# FIG.4

EP 0 177 004 B1

**FIG.6**

**FIG.5**